# EUROPÄISCHE PATENTSCHRIFT

(11) **EP 0 756 827 B1**
(45) Veröffentlichungstag und Bekanntmachung des Hinweises auf die Patenterteilung: **27.11.2002**
(21) Anmeldenummer: 96112251.2
(22) Anmeldetag: 30.07.1996
(51) Int. Cl.: A23L 1/30, A23L 1/305, A61K 31/195, A61K 31/015, A61K 31/20, A61K 31/23

(54) **Präparat zur Ernährung**
Composition for nutrition
Préparation pour l'alimentation

(30) Priorität: 03.08.1995 DE 19528461
(43) Veröffentlichungstag der Anmeldung: 05.02.1997
(73) Patentinhaber: B. BRAUN MELSUNGEN AG, 34212 Melsungen (DE)
(72) Erfinder: Windenband, Albrecht, Dipl.Oec.troph., 34212 Melsungen (DE); Pausch, Gudrun, Dipl.Hum.Biol., 34212 Melsungen (DE); Karsten, Simone, Dr.Oec.troph., 34286 Spangenberg (DE)
(74) Vertreter: Jönsson, Hans-Peter, Dr.Dipl.-Chem.

(56) Entgegenhaltungen:
- EP-A- 0 367 724
- EP-A- 0 611 568
- WO-A-91/09524
- WO-A-93/16595
- PROCEEDINGS OF THE NUTRITION SOCIETY, Bd. 44, 1985, Seiten 221-229, XP000196513 BUDOWSKI P.;ET AL: "Alpha-linolenic acid as a regulator of the metabolism of arachidonic acid: dietary implications of the ratio, n-6:n-3 fatty acids"
- INFUSIONTHERAPIE, Bd. 14, Nr. 3 supplement , 1987, Seiten 20-28, XP002033120 WOLFRAM G.: "Essentielle Fettsäuren in der parenteralen Ernährung"

## Beschreibung

Die Erfindung betrifft Präparate, insbesondere gebrauchsfertige Flüssignahrungen, zur enteralen und/oder oralen Ernährung, insbesondere von Patienten mit geschwächter Immunfunktion oder Tumorpatienten enthaltend Eiweiß, Kohlenhydrate, Fett, Ballaststoffe und Wasser.

Bei Patienten mit verminderter Immunkompetenz, wie beispielsweise bei HIV- oder Tumorpatienten tritt im Verlauf der Erkrankung häufig ein progredienter Gewichtsverlust mit nachfolgender Kachexie oder Anorexie auf. Zu den wesentlichen Folgen der Malnutrition gehören die Verminderung der Lebensqualität, eine erhöhte Morbidität (wie Pneumonie, Hamwegsinfekte, Abzesse, Wundheilungsstörungen) sowie eine Zunahme der Komplikationsrate nach Strahlen- und Chemo-Therapie und nach chirurgischen Eingriffen. Außerdem führt die Katabolie körpereigener Proteine zu deutlichen Funktionsstörungen verschiedener Organe, wie dem Immunsystem und dem Gastrointestinaltrakt.(P. Schauder (1991); Ernährung und Tumorerkrankungen; P. Schauder (Ed.), pp 1-18, Karger Verlag Basel).

Das Auftreten der Kachexie kann unter anderem auf Imbalanzen der Immunfunktionen zurückgeführt werden. Relevant sind in diesem Zusammenhang vor allem einige Cytokine, die von verschiedenen immunkompetenten Zellen synthetisiert werden. Diese Mediatoren können als Modulatoren des Substrat- und Energiestoffwechsels sowie durch Hemmung des Appetits zu einer negativen Energiebilanz beitragen (U. Keller (1991); Ernährung und Tumorerkrankungen; P. Schauder (Ed), pp 213-222, Karger Verlag Basel; O. Selberg, A. Weimann, MJ. Müller (1991): Ernährung und Tumorerkrankungen; P. Schauder (Ed), pp 198-213, Karger Verlag Basel). Die dadurch bedingte Malnutrition kann wiederum eine Verminderung der Immunfunktionen und eine Erhöhung der Infektanfälligkeit hervorrufen. Aus diesen synergistischen Wechselbeziehungen resultiert ein circulus vitiosus.

Eine hohe Fettzufuhr über einen längeren Zeitraum beeinträchtigt die Funktionen des Immunsystems. Umgekehrt kann bei Gesunden und bei Tumorpatienten durch eine Reduzierung des Fettanteils in der Diät auf ca. 25 Energieprozent eine Stimulation verschiedener immunologischer Parameter erzielt werden (J. Barone, JR. Hebert, MM. Reddy (1989): Am J. Clin. Nutr. 50:861-867; DS. Kelly, RM. Dougherty, LB. Branch et al. (1992): Clin Immunol Immunopathol 62:240-244; SN Meydani, AH. Lichtenstein; S. Cornwall et al. (1993: J. Clin. Invest. 92:105-113; BK Garritson, A. Nikaein, MA. Gorman et al. (1994): Symposium Adjuvant Nutrition in cancer treatment, March 1994, San Diego, California (Abstract)). Das Handelsprodukt Supportan®, welches für kachektische Tumorpatienten empfohlen wird, hat einen hohen Fettanteil von 50 Energieprozent. Dieses Produkt bietet daher keine optimalen Voraussetzungen zur Aufrechterhaltung der Immunkompetenz der Patienten.

Handelsübliche Sondennahrungen werden weitgehend auf Basis von Sojabohnenöl hergestellt. Das Fettsäurenmuster im Sojabohnenöl setzt sich zusammen aus: 54 % Linolsäure, 24 % Ölsäure, 11 % Palmitinsäure, 7 % α-Linolensäure, 4 % Stearinsäure. Im Vergleich zur normalen Ernährung ist der Anteil der essentiellen Linolsäure (ω-6-Fettsäure) ausgesprochen hoch, während andere Fettsäuren gar nicht oder nur vergleichsweise gering enthalten sind. Linolsäure wirkt bei hoher Dosierung in vivo hemmend auf verschiedene immunologische Parameter (J. Nordenström, CJ. Arstrand, A. Wiernick (1979): Am. J. Clin. Nutr. 32:2416-2422; I. Fraser, J. Neotolemos, P. Wood et al. (1983): Clin. Nutr. 2:37-40; DMA. Francis, BK. Shenton (1987): Aus. NZ. J. Surg. 57:323-329; JRT. Monson, PC. Sedmann, CW. Ramsden et al. (1988): Eur. J. Surg. Oncol. 14:435-443; CA. Gogos, FE. Kalfarentzos, NC. Zoumbos (1990): Am. J. Clin. Nutr. 51:119-122). Klinische Beobachtungen unterstützen diese Befunde. Zum einen kann nach hoher Linolsäure-Zufuhr eine Zunahme der septischen Komplikationen beobachtet werden (PJ. Guillou (1993): Proc. Nutr. Soc. 52:91-100). Zum anderen wurde Linolsäure erfolgreich im Rahmen einer immunsuppressiven Therapie (z. B. nach Nierentransplantation (MI. McHugh, R. Wilkinson, RW. Elliott et al. (1977): Transplantation 24:263-267), bei Multipler Sklerose (JH. Millar, KJ. Zilkha, MJS. Lanman et al. (1973: Brit. Med. J. 1:765-768) oder bei rheumatioder Athritis (JM. Kremer, AV. Michalek, L. Linninger et al. (1985): Lancet i:184-187) als Adjuvans eingesetzt.

Diese Effekte werden vermutlich über Prostaglandin E2, dem Cyclooxygenaseprodukt der Linolsäure vermittelt. Die oben genannten Effekte der Linolsäure sind bei Patienten mit geschwächter Immunkompetenz unerwünscht. Das Fettsäurenmuster der Sondendiät sollte daher durch ein ausgeglichenes Verhältnis der unterschiedlich strukturierten Fettsäuren an diese Stoffwechselsituation angepaßt sein.

Eicosapentaensäure (EPA) und Docosahexaensäure (DHA) sind ω-3-Fettsäuren. Sie sind in den Handelsprodukten Supportan® und Impact® in einer Dosierung von 0,17 g/100 ml beziehungsweise 0,31 g/100 ml enthalten. Dies entspricht einer täglichen Zufuhr von 2,65 g beziehungsweise 4,65 g bei Einhaltung der empfohlenen Tagesdosis von 1500 ml. Für diese Produkte werden folgende Anwendungsbereiche angegeben: Patienten mit hypermetabolem Zustand mit erhöhtem Infektionsrisiko (Impact®) sowie kachektische Tumorpatienten (Supportan®). Studienergebnisse zeigen allerdings, daß bei einer langfristigen Applikation an EPA und DHA in einer Dosierung von mehr als 1 g pro Tag verschiedene immunologische Funktionen gehemmt werden. Dazu zählt eine Abnahme im CD4/CD8-Verhältnis (SN. Meydani, S. Endres, MM. Woods et al. (1991): Nutr. Immunol. 121:547-555), eine verminderte Reaktion gegenüber dem Recall-Antigen-Hauttest (Meydani loc. cit.), eine verminderte Synthese von Akut-Phase-Proteinen (E. Ernst, T. Saradeth, G. Achhammer (1991): Eur. J. Clin. Invest. 21:77-82; AL. Cooper, L. Gibbons, MA. Horan et al. (1993): Clin. Nutr. 12:321-328), eine Abnahme der Chemotaxis von neutrophilen Lymphocyten (RI. Sperling, AL. Benincaso, CT. Knoell, JK. Larkin (1993): J. Clin. Invest. 91:651-660) sowie eine geringere Cytotoxizität der natürlichen Killerzellen (N. Yamashita, M. Mauyama, K Yamazaki, T. Hamazaki, S. Yano (1991): Clin. Immunol. Immunopath. 59:335-345). Der hohe Gehalt an EPA und DHA in den benannten Produkten ist daher bei den ausgewiesenen Indikationsstellungen von Nachteil.

Aus den mehrfach ungesättigten Fettsäuren der ω-3 und ω-6-Reihe leiten sich jeweils verschiedene Mediatoren ab, die sich in ihrer Struktur und in ihren Wirkprofilen zum Teil deutlich unterscheiden. Da die ungesättigten Fettsäuren bei der Metabolisierung um das gleiche Enzymsystem konkurrieren, sollen die Fettsäuren aus der ω-3 und ω-6 Reihe in einem ausgewogenen Verhältnis zugeführt werden. Empfehlenswert ist ein Verhältnis der ω-3- zu ω-6-Fettsäuren im Gewichtsverhältnis von 1 zu 3 bis 1 zu 7 (P. Budowsky, MA. Crawford (1985): Proc. Nutr. Soc. 44:221-229; G. Wolfram (1987): Infusionsther. 14 (Suppl.3):20-28).

Glutamin ist nach der Definition von Rose (Commonwealth Bureau of Animal Nutrition (1957:631-647) eine nichtessentielle Aminosäure. Dennoch gewinnt sie in der klinischen Ernährung einen zunehmenden Stellenwert, da in Studien die nutritive Bedeutung dieser Aminosäure gezeigt werden konnte (TR. Ziegler et al. (1992) Annals of Internat Medicine, 116:821). Glutamin ist ein wichtiges Energiesubstrat für schnell proliferierende Zellen, wie z. B. für immunkompetente Zellen und Enterocyten. Außerdem ist Glutamin als Aminogruppendonator an der Synthese von RNA und DNA beteiligt und somit für alle Zellteilungen erforderlich.

Daher ist Glutamin ein wichtiger Nährstoff zur Aufrechterhaltung der gastrointestinalen Funktionen. Wie klinische Studien zeigen, verbessert Glutamin die Struktur und Integrität der Darmmucosa (RRWJ. van der Hulst, BK van Kreel, MF. von Meyenfeldt et al. (1993): Lancet. 341:1363-1365; W. Scheppach, C. Oges, P. Bartram, ST. Christl, F. Richter, G. Dusel, P. Stehle, P. Fürst, H. Kasper (1994): Gastroenterology 107, 429-434; H. Tremel, B. Kienle, LS. Weilemann, P. Stehle, P. Fürst (1994): Gastroenterology 107; 1595-1601). Tierexperimentell kann durch orale Glutamingaben die bakterielle Translokation und Sepsis vermindert werden (L. Gianotti et al., J. of Parenteral and Enteral Nutrition (1985), Vol. 19, 60). Außerdem ist Glutamin wichtiges Nährsubstrat für Immunzellen und daher bei Stressituationen wie Trauma, Infektion oder Kachexie vorteilhaft (M.S.W. Ardawi (1985): 21, 1-44). Bei diesen Stoffwechsellagen sind aber die Glutaminspiegel im Blutplasma erniedrigt (J. Askanazi, P. Fürst, CB. Michelsen (1980): Ann. Surg. 191:465-472) und in der Muskulatur ist eine Glutamin-Depletion nachweisbar (MK. Chen, NJ. Espat, KL. Bland et al. (1993): Ann. Surg. 217: 655-667). Diese Aspekte erklären die Vorteile einer glutaminreichen Diät bei den genannten Indikationen. Eine Glutamin-Anreicherung enteraler Flüssignahrungen war bisher nicht möglich, da Glutamin als freie Aminosäure eine große Instabilität in wässriger Lösung aufweist, und die Hitzesterilisation mit der Bildung von verschiedenen Substanzen, wie Pyroglutamat, verbunden, die zum Teil toxisch wirken können. Daher war es bisher schwierig, den Glutamingehalt in den Sondennahrungen auf die besonderen Bedürfnisse der genannten Zielgruppe abzustimmen.

Die Aminosäure Arginin ist unter anderem Ausgangssubstanz für die Synthese von Collagen und Polyaminen. Daher kann Arginin möglicherweise zur Verbesserung der Wundheilung beitragen und das Wachstum, die Teilung und die Differenzierung immunkompetenter Zellen begünstigen. Einen hohen Arginin-Gehalt weist das Handelsprodukt Impact® (1,25 g/100 ml) auf. Bei gesunden Probanden konnte unter dem Zusatz von Arginin zwar ein Anstieg der Collagen-Synthese gezeigt werden (A. Barbul, SA. Lazarou, DT. Efron et al. (1990): Surgery 108: 331-337; SJ. Kirk, M. Hurson, MC. Regan et al. (1993) Surgery 114:155-160), bei Patienten wurde aber keine Verbesserung der Wundheilung nachgewiesen. Auch hinsichtlich der immunologischen Effekte liegen keine eindeutigen Daten vor. Allerdings konnte human- und tierexperimentell durch Arginin eine Zunahme im Tumorwachstum beobachtet werden (VB. Grossie, K Nishioka, JA. Ajani et al. (1992): Surg. Oncol. 50:161-167, K.G.M. Park, S. D. Heys, K. Blessing et. al. (1992): Clin. Sci.: 82: 413-417).

Cystein spielt als Schwefel-haltige Aminosäure und als Bestandteil des Glutathions möglicherweise eine wichtige Rolle als Antioxidanz. Vor diesem Hintergrund wird der therapeutische Einsatz vor allem bei HIV-Infektion diskutiert. Unterstützend sind die Beobachtungen, daß HIV-Patienten einen verminderten Cystein-Spiegel im Blutplasma (R. Buhl, KJ. Holroyd, A. Mastrangeli et al. (1989): Lancet ii: 1294-1298) und einen geringeren intrazellulären Glutathion-Gehalt (HP. Eck, H. Gmunder, M. Hartmann et al. (1989): Biol. Chem. Hoppe-Seyler 370:101-108) aufweisen. Außerdem wurde in Zellkulturversuchen eine Abnahme der HIV-Replikation durch den Zusatz von Cystein gezeigt (M. Roederer, FJT. Staal, PA. Raju et al. (1990): Proc. Natl. Acad. Sci. USA 87:4884-4888); S. Mihm, J. Ennen, U. Pessara et al. (1991) Aids 5:497-503).

Als immunstimulierende Komponente wird der Zusatz von RNA-Hefen in enteralen Diäten diskutiert. Hintergrund dieser Überlegung ist die Tatsache, daß Pyrimidine für das Wachstum und die Proliferation von Zellen erforderlich sind. Daher können Pyrimidin-haltige Nährlösungen möglicherweise die zellulären Immunfunktionen verbessern. RNA-Hefen sind in den Handelsprodukten Impact® (0,12 g/100 ml) und Supportan® (0,13 g/100 ml) enthalten. Der therapeutische Nutzen von RNA-Hefen konnte durch klinische Studien nicht belegt werden. Bei der Diskussion über die therapeutische Effizienz ist zu berücksichtigen, daß maximal 20 % der aufgenommenen RNA für den Intermediärstoffwechsel bioverfügbar sind (15th Congress on Clinical Nutrition and Metabolism 1993, Pharmacists Circle). Außerdem ist zu bedenken, daß durch einen Hefezusatz die für Sondennahrungen gewünschte Purinfreiheit nicht gegeben ist.

Aufgrund der neueren Erkenntnisse über den Radikalstoffwechsel gewinnen die Antioxidantien (Vitamin E, Vitamin C, β-Carotin) sowie Selen als Bestandteil der antioxidativ wirkenden Glutathionperoxidase für die klinische Ernährung zunehmend an Bedeutung. Als Scavenger von sauerstoffreaktiven Substanzen schützen sie die Zellen vor Schädigungen der Membran und der DNA (H. Böhles, Z. Geriatrie (1991) 4:358-372). Außerdem sind sie effektive Stimulatoren der humoralen und zellulären Immunantwort (A. Bendich 1993 Vitamin E and human immune functions. In: Human Nutrition - A comprehensive treatise, Vol. 8, Nutrition and Immunology, Klurfeld DM (Ed.) Plenum Press NY, pp:217-228; A. Roe, CJ. Fuller 1993: Carotinoids and immune function. In: Human Nutrition - A comprehensive treatise, Vol. 8 Nutrition and Immunology, Klurfeld DM. (Ed.) Plenum Press NY, 229-238; BV. Siegel 1993: Vitamin C and the immune response in health and disease. In: Human Nutrition - A comprehensive treatise. Vol.8 Nutrition and Immunology, Klurfeld DM (Ed.) Plenum Press NY, pp: 167-196; JR. Stabel, JW. Spears 1993: Role of selenium in immune responsiveness and disease resistance. In: Human Nutrition - A comprehensive treatise. Vol.8 Nutrition and Immunology; Klurfeld DM (Ed.) Plenum Press NY, pp: 217-228). Für die optimale Wirksamkeit ist die kombinierte Verabreichung dieser Substanzen entscheidend (Trabold 1993 PTA heute 7: 669-672).

Der Zusatz der Vitamine C und E, β-Carotin und Selen ist insbesondere bei Patienten mit geschwächter Immunkompetenz, Tumorpatienten und proinflammatorischen Erkrankungen wichtig, da diese Erkrankungen vielfach mit einer verstärkten Radikalgenese einhergehen, insbesondere während der Strahlen- und Chemotherapie (M.R. Clemens et al. (1994): Einflüsse von Chemo- und Strahlenbelastung auf den Antioxidantienstatus. Vit. Min. Spur. 9: 76-81; MP. Look, E. Musch (1994): Lipid peroxides in the polychemotherapie of cancer patients. Pharmacology 40: 8-15; C. Sappey et. al. (1994) Vitamin, trace element and peroxide status in HIV seropositive patients: asymptomactic patients present a severe beta-carotene deficiency, Clin. Chim. Acta 230: 35-42). Außerdem haben diese Patienten einen schlechten Versorgungsstatus mit Selen und Antioxidantien (D.A. Roe et. al. (1992): Effects of drugs on vitamin needs. Ann NY Acad. Sci 669: 156-164; K. Winnefeld et al. (1995): Der Selen- und Antioxidantienstatus bei verschiedenen Krankheitsbildern. Med. Klin. Suppl. 1:7-10).

WO 93/16 595 beschreibt eine flüssige Emährungsmittelzusammensetzung für Traumaoder chirugische Patienten mit einer Kaloriendichte von etwa 1,2 bis 1,5 kcal/ml und einem Kalorien:Stickstoffverhältnis von etwa 112 : 1 bis 145 : 1. Ein Teil des Proteinsystems umfaßt teilweise verdautes Protein, wobei ein Energiegehalt von 1 bis 3 Engergieprozent des Produktes auf L-Arginin zurückgeht. Das Fettsäurenmuster weist ein Verhältnis von Linolsäure zu α-Linolensäure im Bereich von 3,5 : 1 bis 5,5: 1 auf.

Die EP 0 626 175 A2, EP 0 626 176 A2 und EP 0 626 177 A2 betreffen Präparate zur enteralen Ernährung, insbesondere zur Ernährung von Patienten mit geschwächter Immunfunktion, wobei das Präparat ein Sojaproteinhydrolysat mit einer speziellen Molekulargewichtsverteilung ist, die durch Größenausschlußchromatographie bestimmt wird. 30 bis 60 % der Teilchen haben eine Molekularmasse im Bereich von 1500 bis 5000 Dalton. Das Präparat enthält weiterhin eine Quelle von intaktem Protein. Das Fettsäurenmuster weist ein Verhältnis von ω-6-Fettsäuren zu ω-3-Fettsäuren von etwa 1,3 bis 2,5 : 1 auf.

Die EP 0 611 568 A1 beschreibt Präparate zur enteralen Ernährung, insbesondere von onkologischen Patienten, wobei die Präparate Fette und gegebenenfalls Kohlenhydrate und/oder Proteine sowie gegebenenfalls andere übliche Nährstoffe, Hilfsstoffe und Zusatzstoffe enthalten. Diese Präparate sind durch den hohen Fettgehalt und das spezielle Verhältnis der ω-3-Fettsäuren zu den ω-6-Fettsäuren charakterisiert. Die Fettkomponente der hier beschriebenen Präparate weist das folgende Fettsäurenmuster, angegeben in Gewichtsprozent, bezogen auf den Gesamtfettgehalt auf, wobei die Fettsäuren sowohl in freier Form als auch in Form von verträglichen Salzen und/oder Estern vorliegen können:

| | |
|---|---|
| Ölsäure | 30 - 55 Gew.-% |
| Linolsäure | 3 - 20 Gew.-% |
| α-Linolensäure | 0,5 - 8 Gew.-% |
| Eicosapentaensäure und Docosahexaensäure zusammen | 1 - 10 Gew.-% und |
| sonstige ω-3-Fettsäuren | 0 - 0,5 Gew.-%, |

wobei das Verhältnis der ω-3-Fettsäuren zu den ω-6-Fettsäuren im Bereich von 1 : 2,1 bis 1 : 3 liegt. In den hier beschriebenen Präparaten kann, bezogen auf das gesamte Präparat, der Fettgehalt 40 - 65 Energieprozent, der Proteingehalt 12 - 25 Energieprozent und der Kohlenhydratgehalt 20- 45 Energieprozent ausmachen. Die Fettkomponente kann auch MCT enthalten.

In der WO 87/01589 wird ein Verfahren zur Behandlung von katabolischen Fehlfunktionen beschrieben, wobei die verabreichte Zusammensetzung eine therapeutisch wirksame Menge von Glutamin oder einem Analogon enthält.

In der EP 0 367 724 B1 werden immunstimulierende Zusammensetzungen beschrieben, die in einer Einheitsdosis enthalten: 3 bis 40 g Arginin oder eine äquivalente Menge eines Argininvorläufers oder Ornithin, 0,1 bis 4,0 g RNA oder eine äquivalente Menge einer anderen Nukleotidbasenquelle, 0,1 bis 20 g ω-3-polyungesättigte Fettsäuren und 0,1 bis 20 g ω-6-polyungesättigte Fettsäuren.

Die WO 91/09524 betrifft die Behandlung von Humanpatienten bezüglich der Immunantwort oder zur Reduktion der Schwere des Abbaus der Humanimmunreaktion durch Verabreichung von Arginin oder Ornitin oder einem funktionellen Analogon dieser Aminosäuren.

Die Aufgabe der vorliegenden Erfindung besteht in der Bereitstellung eines verbesserten Präparates zur enteralen und/oder oralen Ernährung, insbesondere von Patienten mit geschwächter Immunfunktion oder Tumorpatienten, das diesen Indikationen besondere Aufmerksamkeit schenkt und dabei insbesondere die Verstoffwechselung verschiedener Fette und/oder Fettsäuren, deren Wirkung auf immunologische Vorgänge und/oder damit verbundene Prozesse und Krankheiten berücksichtigt. Darüber hinaus galt es, die Rolle von speziellen Aminosäuren im Stoffwechsel, insbesondere Glutamin, Arginin und Cystein sowie ebenfalls deren Wirkung auf immunologische Funktionen und damit verbundene Prozesse und Krankheiten sowie Stabilität bei oraler Gabe zu berücksichtigen.

Weiterhin bestand die Aufgabe der vorliegenden Erfindung darin, die Verstoffwechselung von RNA-Hefen sowie deren Wirkung auf metabolische Vorgänge und die damit verbundenen Prozesse und Krankheiten zu berücksichtigen. Eine weitere Aufgabenstellung der vorliegenden Erfindung ergibt sich aus der funktionalen Bedeutung von Antioxidantien und Selen im Intermediär-Stoffwechsel sowie ebenfalls deren Wirkung auf immunologische Vorgänge und damit verbundene Prozesse und Krankheiten sowie deren Bioverfügbarkeit bei oraler Gabe.

In einer ersten Ausführungsform der vorliegenden Erfindung wird die Hauptaufgabe gelöst durch ein Präparat, insbesondere Flüssignahrung, zur enteralen und/oder oralen Ernährung, insbesondere von Patienten mit geschwächter Immunfunktion oder Tumorpatienten, enthaltend Eiweiß und/oder Eiweißhydrolysate, Kohlenhydrate, Fett, Ballaststoffe und Wasser mit einem Fettgehalt von 20 bis 30 Energieprozent, wobei das Fettsäurenmuster
(a) einen MCT-Anteil von 30 - 70 %
(b) ein Verhältnis der ω-3-Fettsäuren zu ω-6-Fettsäuren von 1 zu 3,1 bis 1 zu 7
(c) ein Verhältnis der ω-6-Fettsäuren zu ω-9-Fettsäuren von 1 zu 0,7 bis 1 zu 1,4 und
(d) ein Verhältnis der einfach ungesättigten Fettsäuren zu den mehrfach ungesättigten Fettsäuren von 1 zu 0,5 bis 1 zu 1,5 aufweist, wobei die Eiweißkomponente 0,5 bis 3,0g Glutamin pro 100 ml gebrauchsfertiger Lösung umfaßt.

Mit Hilfe der vorliegenden Erfindung wird eine vollständig bilanzierte Sondennahrung, die zur ausschließlichen und ergänzenden enteralen und/oder oralen Ernährung bestimmt ist, zur Verfügung gestellt. Die Nährstoffzusammensetzung dieser Diät ist speziell auf den Bedarf von Patienten mit geschwächter Abwehrlage abgestimmt. Daher ist die Anwendung der erfindungsgemäßen Präparate vor allem im Rahmen der Ernährungstherapie von Tumor-, Aids- und Streßpatienten, Patienten in der postoperativen Phase, septischen Patienten, Patienten mit proinflammatorischem Entzündungsgeschehen, Patienten mit entzündlichen Darmerkrankungen sowie bei Trauma und Verbrennungen geeignet.

Die erfindungsgemäßen Präparate weisen einen Fettgehalt von 20 bis 30 Energieprozent, insbesondere 25 Energieprozent auf, der optimale Voraussetzungen zur Aufrechterhaltung der Immunkompetenz der Patienten bietet.

Der MCT-Anteil des Fettsäurenmusters beträgt erfindungsgemäß 30 bis 70 % und insbesondere 30 bis 50 %. Mittelkettige Fettsäuren haben gegenüber den langkettigen Fettsäuren eine deutlich geringere immunsupressive Wirkung und sind daher als Energiesubstrat gut geeignet(W.A. Gogos et al. (1994), J.Am.Coll.Nutr. 13:40-44, Sedman et al. (1991) Br. J. Surg. 78:1396-1399). Geringe Mengen an MCT-Fettsäuren sind weniger geeignet, da sonst der immunologische Effekt nicht zum Tragen kommt. Höhere Mengen sind ebenfalls nicht geeignet, da bei einem Fettanteil von 20-30 Energieprozent der Bedarf an essentiellen Fettsäuren nicht gedeckt werden kann.

Der Gehalt an Eicosapentaensäure und Docosahexaensäure in den erfindungsgemäßen Präparaten ist gegenüber im Handel erhältlichen Produkten Impact® oder Supportan® deutlich vermindert und entspricht lediglich 0,05 bis 1 g pro 100 ml entsprechend einer Tagesdosis von etwa 1 g.

Erfindungsgemäß wird das Verhältnis der ω-3-Fettsäuren zu ω-6-Fettsäuren im Bereich von 1 : 3,1 bis 1 : 7, vorzugsweise im Bereich von 1 : 3,1 bis 1 : 5 eingestellt, um ein ausgewogenes Verhältnis der ω-3- zu den ω-6-Fettsäuren zu erhalten.

Neben dem Verhältnis der ω-3-Fettsäuren zu ω-6-Fettsäuren ist das Verhältnis der ω-6-Fettsäuren zu den ω-9-Fettsäuren erfindungsgemäß von besonderer Bedeutung. Hierbei ist wesentlich, daß das Verhältnis von ω-6-Fettsäuren zu ω-9-Fettsäuren im Bereich von 1 : 0,7 bis 1 : 1,4 eingestellt wird, da die ungesättigten Fettsäuren bei der Metabolisierung um das gleiche Enzymsystem konkurrieren. Damit diese Stoffwechselwege in gleicher Intensität ablaufen können, ist eine ausgewogene Zufuhr dieser Fettsäuren wichtig.

Auch das Verhältnis der einfach ungesättigten Fettsäuren zu mehrfach ungesättigten Fettsäuren ist erfindungsgemäß von besonderer Bedeutung und wird auf dem Bereich von 1: 0,5 bis 1 : 1,5 eingestellt, da nur unter diesen Bedingungen das gewünschte ausgeglichene Verhältnis der ω-3-Fettsäuren zu ω-6-Fettsäuren und das Verhältnis der ω-6-Fettsäuren zu ω-9-Fettsäuren gewährleistet werden kann.

Besonders bevorzugt im Sinne der vorliegenden Erfindung ist ein Präparat, bei dem das Fettsäurenmuster durch Mischung von MCT-Öl, Rüböl, Borretschöl, Nachtkerzenöl und/oder Fischöl eingestellt wird. Mit den genannten Ölen ist es ohne weiteres möglich, die obengenannten Verhältnisse der verschiedenen Säuren untereinander einzustellen.

Dabei enthält das erfindungsgemäße Präparat 0,5 bis 3 g, insbesondere 0,7 bis 2g/100ml Glutamin. Der Glutamingehalt der erfindungsgemäßen Sondennahrungen ist auf die besonderen Bedürfnisse der genannten Zielgruppe abgestimmt. Für das erfindungsgemäße Präparat wird insbesondere ein Glutamin-angereichertes Proteinhydrolysat verwendet, das in dieser Formulierung auch während des Herstellungsverfahrens seine Stabilität erhält und aus dem keine toxischen Produke hervorgehen. Der Glutamingehalt in den erfindungsgemäßen Präparaten entspricht einer Tagesdosis von durchschnittlich 16 g.

Besonders bevorzugt im Sinne der vorliegenden Erfindung ist ein Glutamingehalt von 1 bis 3 g/100 ml. Bei höherer Dosierung besteht das Risiko einer Aminosäure-Imbalanz.

In einer weiteren Ausführungsform der vorliegenden Erfindung enthalten die erfindungsgemäßen Präparate Antioxidantien, ausgewählt aus Vitamin E, Vitamin C, b-Carotin und/oder Selen. Besonders bevorzugt ist es, die Substanzen in einer Dosierung mit metabolischer Wirkung einzusetzen, wobei gemäß der vorliegenden Erfindung die Mengenobergrenzen an den zur Zeit in Deutschland vorgegebenen maximal zulässigen Tagesmengen der Diätverordnung angesiedelt sind. Die bevorzugten Mindestmengen sind erforderlich, um die Wirkung zu entfalten.

In einer weiteren Ausführungsform der vorliegenden Erfindung ist es von besonderer Bedeutung, daß das Präparat keinen Zusatz von Arginin enthält. Auch in diesem Fall ist jedoch das Präparat nicht notwendigerweise Arginin-frei, da Arginin als Bestandteil von Proteinen und Proteinhydrolysaten vorkommt und somit bei Verwendung dieser Bestandteile ein inhärenter Argininanteil in dem Präparat enthalten ist. Dementsprechend ist eine Anreicherung mit Arginin nicht empfehlenswert. Dies entspricht auch dem Konsens der Panel Discussion "Arginin-Supplementation in der enteralen und parenteralen Ernährung, Facts and Fiction" anläßlich der Jahrestagung der DGEM und AKE im März 1994.

Zum Erhalt von purinfreier Sondennahrung ist es im Sinne der vorliegenden Erfindung bevorzugt, keine RNA-Hefen einzusetzen.

In einer weiteren bevorzugten Ausführungsform der vorliegenden Erfindung ist das Präparat vorzugsweise durch einen Cysteingehalt von 30 bis 400 mg/100 ml gekennzeichnet. Dieser Konzentrationsbereich kann einerseits einen therapeutischen Effekt auslösen, andererseits wird durch die Begrenzung von 400 mg/100 ml dem Risiko einer Aminosäure-Imbalanz vorgebeugt.

γ-Linolensäure ist eine Fettsäure der ω-6-Familie. Sie zeigt ein anderes immunologisches Wirkprofil als Linolsäure. Im Intermediärstoffwechsel wird γ-Linolensäure in Di-Homo-γ-Linolensäure metabolisiert, der Ausgangssubstanz von Prostaglandin E1. Dieses Cyclooxygenaseprodukt zeigt im Vergleich zu PGE2 eine deutlich geringere immunsuppressive Wirkung (Kinsella JE und Lokesh B (1990) Crit. Care Med. 18:94-113). Vor diesem Hintergrund lassen sich die positiven Effekte einer γ-Linolensäure-reichen Fettzufuhr auf das Immunsystem bei verschiedenen Patientengruppen, Patienten mit HIV-Infektion (Horrobin DF (1992), Prog. Lipid Res. 31:163-194), Tumorpatienten (Falconer JS et al. (1993): Proc. Nutr. Soc. 52: 244A und van der Merwe CF et al. (1987): Br. J. Clin. Pract. 41:907-915) sowie Patienten mit proinflammatorischem Entzündungsgeschehen (Belch JJ et al. (1988): Annu. Rheum. Dis. 47:96-104, Hansen TM et al. (1983): Scand J. Rheumatol 12:85-88 und Horrobin DF (1992), Prog. Lipid Res. 31:163-194) erklären.

Gegenüber den im Handel erhältlichen Produkten Impact® oder Supportan® wurden den erfindungsgemäßen Präparaten insbesondere 0,07 g g-Linolensäure/100 ml zugesetzt. Eine weitere Ausführungsform der vorliegenden Erfindung besteht in der Verwendung von Präparaten wie oben definiert zur **Herstellung von Sondenoder Flüssignahrung, insbesondere gebrauchsfertiger Flüssignahrung zur** Ernährung von Patienten mit geschwächter Immunfunktion, Tumorpatienten, Patienten mit HIV-Infektion, Patienten in der postoperativen Phase, septischen Patienten, Patienten mit proinflammatorischen Entzündungsgeschehen oder Patienten mit entzündlichen Darmerkrankungen.

Das nachfolgende Ausführungsbeispiel gibt eine geeignete Rezeptur beziehungsweise die Zusammensetzung eines erfindungsgemäßen Präparats wieder.

| Ausführungsbeispiel | |
|---|---|
| Bilanzierte Diät für Heranwachsende und Erwachsene | |
| Zusammensetzung Angaben pro 100 ml | |
| Wasser | 77,00 ml |
| Maltodextrin | 17,79 g |
| hydrolysiertes Milcheiweiß | 4,52 g |
| hydrolysiertes Pflanzeneiweiß (Weizen) | 3,62 g |
| Sojaballaststoff | 1,78 g |
| pflanzliche Öle *) | 1,61 g |
| MCT-Öl | 1,48 g |
| Stärke | 0,50 g |
| Emulgator: Lecithin | 0,25 g |
| Natriumchlorid | 0,23 g |
| di-Kaliumhydrogenphosphat | 0,17 g |
| tri-Kaliumcitrat H₂O | 0,14 g |
| Fischöl | 0,13 g |
| tri-Natriumcitrat 2 H₂O | 0,09 g |
| Magnesiumcarbonat | 0,06 g |
| Calciumhydrogenphosphat | 0,03 g |
| tri-Magnesiumcitrat wfr.(wasserfrei) | 0,03 g |
| tri-Calciumcitrat 4 H₂O | 0,01 g |
| Vitamin C | 8,00 mg |
| Eisen-III-pyrophosphat | 3,10 mg |

| | |
|---|---|
| *) Pflanzliche Öle: Rüböl 1,184 g Borretschöl 0,165 g Nachtkerzenöl 0,259 g | |

| Fortsetzung: | |
|---|---|
| Bilanzierte Diät für Heranwachsende und Erwachsene | |
| Zusammensetzung Angaben pro 100 ml | |
| Zinksulfat H₂O | 2,74 mg |
| Vitamin E | 1,60 mg |
| Nicotinsäureamid | 1,47 mg |
| Calcium-Pantothenat | 1,00 mg |
| Kupfersulfat 5 H₂O | 0,99 mg |
| Mangan-II-chlorid 4 H₂O | 0.96 mg |
| Natriumfluorid | 0.35 mg |
| Vitamin B2 | 0,20 mg |
| Vitamin B6 | 0,20 mg |
| Vitamin 81 | 0,16 mg |
| β-Carotin | 0,13 mg |
| Vitamin A | 0,12 mg |
| Chrom-III-chlorid 6 H₂O | 40,99 µg |
| Natriummolybdat 2 H₂O | 32,80 µg |
| Natriumselenit 5 H₂O | 22,20 µg |
| Kaliumiodid | 20,90 µg |
| Folsäure | 20,00 µg |
| Btotin | 9,00 µg |
| Vitamin K1 | 5,33 µg |
| Vitamin D3 | 0,84 µg |
| Vitamin B12 | 0,60 µg |

| Typanalyse | | |
|---|---|---|
| Angaben pro 100 ml | | |
| Energie | kcal | 133 |
| Protein | g | 6,67 |
| Glutamin | g | 1,07 |
| Kohlenhydrate | g | 18,33 |
| Fett | g | 3,70 |
| MCT | g | 1,48 |
| Ölsäure | g | 0,95 |
| Linolsäure | g | 0,69 |
| Linolensäuren | g | 0,21 |
| alpha-Linolensäure | g | 0,14 |
| gamma-Linolensäure | g | 0,07 |
| EPA/DHA | g | 0,07 |
| Ballaststoffe | g | 1,33 |
| Wasser | ml | 77,00 |
| Osmolalität | mosm/kg | 520 |
| Osmolarität | mosm/l | 400 |
| Natrium | mg | 146,60 |
| Kalium | mg | 226,60 |
| Calcium | mg | 66,70 |
| Magnesium | mg | 26,70 |
| Phosphor | mg | 66,60 |
| Chlorid | mg | 146,60 |
| Eisen | mg | 1,40 |

| Fortsetzung Typanalyse | | |
|---|---|---|
| Angaben pro 100 ml | | |
| Zink | mg | 1,000 |
| Kupfer | mg | 0,250 |
| Iod | mg | 0,016 |
| Chrom | mg | 0,008 |
| Fluorid | mg | 0,160 |
| Mangan | mg | 0,27 |
| Molybdän | mg | 0,013 |
| Selen | mcg | 6,660 |
| Vitamine | | |
| A | mg | 0,120 |
| D | mcg | 0,840 |
| E | mg | 1,600 |
| K | mcg | 5,330 |
| B1 | mg | 0,160 |
| B2 | mg | 0,200 |
| B6 | mg | 0,200 |
| B12 | mcg | 0,600 |
| C | mg | 8,000 |
| Niacin | mg | 1,460 |
| Folsäure | mg | 0,020 |
| Pantothensäure | mg | 1,000 |
| Biotin | mg | 0,009 |
| beta-Carotin | mg | 0,13 |

Die im Ausführungsbeispiel genannte Zusammensetzung zeichnet sich insbesondere durch eine gute Verträglichkeit bei kritisch Kranken aus und ist bei Patienten mit starken Diarrhoen vorteilhaft, wie nachfolgende Kasuistik zeigt.

Eine Patientin mit Schädel-Hirn-Trauma III. Grades mit posttraumatischem Hydrocephalus wurde zur postprimären Rehabilitation auf eine neurologische Schwerpunktstation verlegt. Zum Zeitpunkt der Aufnahme betrug das Körpergewicht der Patientin 45,3 kg bei einer Körpergröße von 1,72. Die Patientin mußte enteral ernährt werden. Sie erhielt über die perkutane endoskopische Gastrostomie (PEG) eine Ballaststoff- und MCT-haltige Sondenernährung.

Wegen rezidivierender Bronchopneumonien wurde die Patientin für 16 Tage mit einem Antibiotikum therapiert. Nach einer Behandlungsdauer von 13 Tagen traten Diarrhoen, teilweise in Form von wäßrigen Stühlen auf. Auch nach Absetzen des Antibiotikums konnten die Stuhlfrequenz und Stuhlkonsistenz nicht normalisiert werden. Einen Monat nach Absetzen des Antibiotikums wurden 3-4 wäßrige Stuhlgänge pro Tag verzeichnet. Diese Darmresorptionsstörung war mit einer Abnahme des Körpergewichts auf 43,8 kg verbunden. Die katabole Stoffwechsellage der Patientin spiegelte sich auch in den pathologischen Gesamteiweißwerten im Blutplasma (6,1 g/dl) wieder. In Stuhluntersuchungen wurden keine darmpathogene Keime nachgewiesen. Daher konnte eine bakteriell-induzierte Diarrhoe ausgeschlossen werden.

In Folge wurde die enterale Ernährung auf das im Ausführungsbeispiel genannte Präparat umgestellt. Bereits nach 9 Tagen wurde eine Normalisierung der Stuhlfrequenz und -konsistenz beobachtet. Nach 15 Tagen war die Verwendung der ursprünglich verwendeten Sondennahrung wieder möglich. Parallel hierzu zeigte sich bei der Patientin eine kontinuierliche Gewichtszunahme. Unter der Behandlung mit dem genannten Präparat konnte das Körpergewicht innerhalb von sieben Tagen um 1,8 kg gesteigert werden.

## Patentansprüche

1. Präparat, insbesondere Flüssignahrung zur enteralen und/oder oralen Ernährung, insbesondere von Patienten mit geschwächter Immunfunktion oder Tumorpatienten, enthaltend Eiweiß und/oder Eiweißhydrolysate, Kohlenhydrate, Fett, Ballaststoffe und Wasser mit einem Fettgehalt von 20 bis 30 Energieprozent, wobei das Fettsäurenmuster
(a) einen MCT-Anteil von 30 - 70 %
(b) ein Verhältnis der ω-3-Fettsäuren zu w-6-Fettsäuren von 1 zu 3,1 bis 1 zu 7
(c) ein Verhältnis der ω-6-Fettsäuren zu w-9-Fettsäuren von 1 zu 0,7 bis 1 zu 1,4 und
(d) ein Verhältnis der einfach ungesättigten Fettsäuren zu den mehrfach ungesättigten Fettsäuren von 1 zu 0,5 bis 1 zu 1,5 aufweist, wobei die Eiweißkomponente 0,5 - 3,0 g Glutamin pro 100 ml gebrauchsfertiger Lösung umfaßt.

2. Präparat nach Anspruch **1**, **dadurch gekennzeichnet, daß** das Fettsäurenmuster ein Verhältnis der ω-3-Fettsäuren zu w-6-Fettsäuren im Bereich von 1 : 3,1 bis 1 : 5 aufweist.

3. Präparat nach Anspruch **1** oder **2**, **dadurch gekennzeichnet, daß** das Fettsäurenmuster durch Mischung von MCT-Öl, Rüböl, Borretschöl, Nachtkerzenöl und/oder Fischöl eingestellt wird.

4. Präparat nach Anspruch **1**, **dadurch gekennzeichnet, daß** die Eiweißkomponente 0,7 bis 2 g Glutamin pro 100 ml gebrauchsfertiger Lösung umfasst.

5. Präparat nach einem oder mehreren der Ansprüche **1** bis **4, dadurch gekennzeichnet, daß** Glutamin in Form von glutaminreichen Proteinhydrolysaten vorliegt.

6. Präparat nach einem oder mehreren der Ansprüche **1** bis **5**, **dadurch gekennzeichnet, daß** es Vitamin E, Vitamin C, beta-Carotin und/oder Selen enthält.

7. Präparat nach Anspruch **6**, **dadurch gekennzeichnet, daß** es 1 bis 5 mg Vitamin E pro 100 ml gebrauchsfertiger Lösung, insbesondere 1-3 mg enthält.

8. Präparat nach Anspruch **6**, **dadurch gekennzeichnet, daß** es 6 bis 30 mg Vitamin C pro 100 ml gebrauchsfertiger Lösung, insbesondere 6 bis 15 mg enthält.

9. Präparat nach Anspruch **6, dadurch gekennzeichnet, daß** es 0,1 bis 1 mg beta-Carotin pro 100 ml gebrauchsfertiger Lösung enthält.

10. Präparat nach Anspruch **6, dadurch gekennzeichnet, daß** es 2 bis 15 µg Selen pro 100 ml gebrauchsfertiger Lösung, insbesondere 4 bis 10 µg enthält.

11. Präparat nach einem oder mehreren der Ansprüche **1** bis **10, dadurch gekennzeichnet, daß** es keinen Zusatz von Arginin enthält.

12. Präparat nach einem oder mehreren der Ansprüche **1** bis **11**, **dadurch gekennzeichnet, daß** es keine RNA-Hefen enthält.

13. Präparat nach einem oder mehreren der Ansprüche **1** bis **12**, **dadurch gekennzeichnet, daß** es einen Cysteingehalt von 30-400 mg pro 100 ml gebrauchsfertiger Lösung aufweist.

14. Verwendung von Präparaten nach einem oder mehreren der Ansprüche **1** bis **13** zur **Herstellung von Sonden- und Flüssignahrung, insbesondere von gebrauchsfertiger Flüssignahrung zur** Ernährung von Patienten mit geschwächter Immunfunktion, Tumorpatienten, Patienten mit HIV-Infektion, Patienten in der postoperativen Phase, septischen Patienten, Patienten mit proinflammatorischem Entzündungsgeschehen und/oder Patienten mit entzündlichen Darmerkrankungen.

## Claims

1. A preparation, in particular liquid diet, for enteral and/or oral nutrition, in particular of patients with impaired immune function or tumor patients, containing proteins and/or protein hydrolysates, carbohydrates, lipids, bulkages, and water, the lipid content being from 20 to 30 energy percent, wherein the fatty acid pattern includes
(a) an MCT content of from 30 to 70%;
(b) a ratio of ω-3 to ω-6 fatty acids of from 1:3.1 to 1:7;
(c) a ratio of ω-6 to ω-9 fatty acids of from 1:0.7 to 1:1.4; and
(d) a ratio of monounsaturated to polyunsaturated fatty acids of from 1:0.5 to 1:1.5;
said protein component comprising from 0.5 to 3.0 g of glutamine per 100 ml of ready-to-use solution.

2. The preparation according to claim 1, **characterized in that** said fatty acid pattern includes a ratio of ω-3 to ω-6 fatty acids in the range of from 1:3.1 to 1:5.

3. The preparation according to claim 1 or 2, **characterized in that** said fatty acid pattern is adjusted by mixing MCT oil, rape-seed oil, borage oil, evening primrose oil and/or fish oil.

4. The preparation according to claim 1, **characterized in that** said protein component comprises from 0.7 to 2 g of glutamine per 100 ml of ready-to-use solution.

5. The preparation according to one or more of claims 1 to 4, **characterized in that** glutamine is present in the form of glutamine-rich protein hydrolysates.

6. The preparation according to one or more of claims 1 to 5, **characterized by** containing vitamin E, vitamin C, β-carotene and/or selenium.

7. The preparation according to claim 6, **characterized by** containing from 1 to 5 mg of vitamin E per 100 ml of ready-to-use solution, in particular from 1 to 3 mg.

8. The preparation according to claim 6, **characterized by** containing from 6 to 30 mg of vitamin C per 100 ml of ready-to-use solution, in particular from 6 to 15 mg.

9. The preparation according to claim 6, **characterized by** containing from 0.1 to 1 mg of β-carotene per 100 ml of ready-to-use solution.

10. The preparation according to claim 6, **characterized by** containing from 2 to 15 µg of selenium per 100 ml of ready-to-use solution, in particular from 4 to 10 µg.

11. The preparation according to one or more of claims 1 to 10, **characterized by** containing no supplement of arginine.

12. The preparation according to one or more of claims 1 to 11, **characterized by** containing no RNA yeasts.

13. The preparation according to one or more of claims 1 to 12, **characterized by** a cysteine content of from 30 to 400 mg per 100 ml of ready-to-use solution.

14. Use of preparations according to one or more of claims 1 to 13 for the preparation of enteral and liquid diets, especially ready-to-use liquid diets, for the nutrition of patients with impaired immune function, tumor patients, patients with HIV infection, patients in the postoperative phase, septic patients, patients with proinflammatory conditions, and/or patients with inflammatory intestine diseases.

## Revendications

1. Préparation, en particulier aliments liquides pour l'alimentation entérale et/ou orale, en particulier de patients ayant une fonction immunitaire affaiblie ou des patients présentant une tumeur, contenant des protéines et/ou des hydrolysats de protéines, des hydrates de carbone, des matières grasses, des charges inertes et de l'eau, avec une teneur en matières grasses de 20 à 30 % en énergie, l'échantillon d'acides gras présentant
a) une proportion de MCT de 30 à 70 %,
b) un rapport entre les acides gras ω-3 et les acides gras ω-6 de 1 : 3,1 à 1 : 7,
c) un rapport des acides gras ω-6 aux acides gras ω-9 de 1 : 0,7 à 1 : 1,4, et
d) un rapport des acides gras monoinsaturés aux acides gras polyinsaturés de 1 : 0,5 à 1 : 1,5, le composant protéinique contenant de 0,5 à 3,0 g de glutamine pour 100 g de solution prête à l'emploi.

2. Préparation selon la revendication 1, **caractérisée en ce que** l'échantillon d'acides gras présente un rapport des acides gras ω-3 aux acides gras ω-6 dans la plage de 1 : 3, 1 à 1 : 5.

3. Préparation selon la revendication 1 ou 2, **caractérisée en ce que** l'échantillon d'acides gras est ajusté par mélange d'huile de MCT, d'huile de colza, d'huile de bourrache, d'huile d'onagre et/ou d'huile de poisson.

4. Préparation selon la revendication 1, **caractérisée en ce que** le composant protéinique contient de 0,7 à 2 g de glutamine pour 100 ml de solution prête à l'emploi.

5. Préparation selon une ou plusieurs des revendications 1 à 4, **caractérisée en ce que** de la glutamine est présente sous forme d'hydrolysats de protéines, riches en glutamine.

6. Préparation selon une ou plusieurs des revendications 1 à 5, **caractérisée en ce qu'**elle contient de la vitamine E, de la vitamine C, du β-carotène et/ou du sélénium.

7. Préparation selon la revendication 6, **caractérisée en ce qu'**elle contient de 1 à 5 mg de vitamine E pour 100 ml de solution prête à l'emploi, en particulier de 1 à 3 mg.

8. Préparation selon la revendication 6, **caractérisée en ce qu'**elle contient de 6 à 30 mg de vitamine C pour 100 ml de solution prête à l'emploi, en particulier de 6 à 15 mg.

9. Préparation selon la revendication 6, **caractérisée en ce qu'**elle contient de 0,1 à 1 mg de β-carotène pour 100 ml de solution prête à l'emploi.

10. Préparation selon la revendication 6, **caractérisée en ce qu'**elle contient de 2 à 15 µg de sélénium pour 100 ml de solution prête à l'emploi, en particulier de 4 à 10 µg.

11. Préparation selon une ou plusieurs des revendications 1 à 10, **caractérisée en ce qu'**elle ne contient pas d'arginine ajoutée.

12. Préparation selon une ou plusieurs des revendications 1 à 11, **caractérisée en ce qu'**elle ne contient pas d'ARN de levure

13. Préparation selon une ou plusieurs des revendications 1 à 12, **caractérisée en ce qu'**elle présente une teneur en cystéine de 30 à 400 mg pour 100 ml de solution prête à l'emploi.

14. Utilisation de préparations selon une ou plusieurs des revendications 1 à 13 pour préparer un aliment pour absorption par sonde et un aliment liquide, en particulier un aliment liquide prêt à l'emploi, pour nourrir des patients ayant une fonction immunitaire affaiblie, des patients présentant une tumeur, des patients infectés par VIH, des patients en phase post-opératoire, des patients qui sont l'objet d'un accident septique, des patients ayant des phénomènes inflammatoires pro-inflammatoires, et/ou des patients ayant des maladies inflammatoires de l'intestin.
